# EUROPEAN PATENT APPLICATION

(11) **EP 3 524 668 A1**
(43) Date of publication of application: **14.08.2019**
(21) Application number: 17857814.2
(22) Date of filing: 22.09.2017
(51) Int. Cl.: C12M 1/36, C12M 1/12, C12N 5/09

(54) **APPARATUS FOR HIGH-THROUGHPUT RAPID TRAPPING OF CIRCULATING TUMOR CELLS AND METHOD FOR PURIFYING CIRCULATING TUMOR CELLS**

(30) Priority: 09.10.2016 CN 201610881083; 15.12.2016 CN 201611157990
(71) Applicant: Shanghai Dida Biotechnology Co., Ltd., Shanghai 200433 (CN)
(72) Inventor: Chen, Jing, Baoshan District Shanghai 200000 (CN)
(74) Representative: Cabinet Laurent & Charras
(86) International application number: PCT/CN2017/102823
(87) International publication number: WO 2018/064933

(57) **Abstract**

An apparatus for high-throughput rapid trapping and purifying of circulating tumor cells and a method for purifying circulating tumor cells, relating to the field of biotechnology. The apparatus for high-throughput rapid trapping of circulating tumor cells comprises: a filter module, the filter module comprises a filter, a filter membrane which is arranged inside the filter and sealed with an inner wall of the filter, and a negative pressure device; the negative pressure device is connected with an end of an outlet of the filter, and used for maintaining a negative pressure within the filter; when a negative pressure is maintained within the filter by the negative pressure device, the filter membrane is configured to trap circulating tumor cells, then stain and wash the trapped cells by using reagents which are automatically loaded by means of a reagent loading module. The apparatus may automatically control loading a sample liquid to the filter as well as the negative pressure device, such that the process in which the circulating tumor cells are trapped by using the filter membrane and then stained has the advantages of being highly automated and having a high throughput (which can be achieved by means of increasing the number of filters).

## Description

The present application claims the priorities of Chinese Patent Application No. CN201610881083.9, entitled "An Apparatus and a Method for High-Throughput Rapid Trapping of Circulating Tumor Cells", filed on October 09th, 2016, and Chinese Patent Application No. CN201611157990.5, entitled "An Apparatus and a Method for High-Throughput Rapid Trapping of Circulating Tumor Cells", filed on December 15th, 2016 to the Chinese Patent Office, which are hereby incorporated herein by reference in their entirety.

### Technical field

The present invention relates to the field of biotechnology, and specifically to an apparatus and a method for high-throughput rapid trapping and purifying of circulating tumor cells.

### Background

Circulating Tumor Cell (CTC) is a general term for various types of tumor cells present in peripheral blood. During the development of tumors, falling off solid tumor lesions (primary lesions, metastases) due to spontaneous or diagnostic operation, most of CTCs are apoptotic or phagocytized after entering peripheral blood, while a minority is capable of escaping and anchoring to develop into a metastatic lesion, which increases the risk of death in patients with malignant tumors and plays an important role in the invasive metastasis of tumors.

Circulating tumor cells shed and enter the circulatory system, which has potential value in aspects such as diagnosis and prognosis of tumor metastasis, drug development, individualized treatment and exploration of tumor metastasis mechanisms, as confirmed by many studies. Therefore, it is very important to detect circulating tumor cells specifically and sensitively, which allows not only more accurate assessment of the prognosis of tumor patients but also the establishment of personalized treatment plans.

According to the pathway of tumor cell metastasis, tumor cells circulating in blood or lymphatic vessels are defined as circulating tumor cells (CTC), wherein the cell clump formed by aggregation of several cells is called circulating tumor mocroemboli (CTM). CTM is a "collective migration" behavior of tumor cells, which has a higher metastatic potential as being capable of resisting cell apoptosis and maintain cell proliferation. However, the number of circulating tumor cells in the peripheral blood is relatively small (1 CTC/106-107 monocytes) and difficult to accurately separate.

The CellSearch system is the only technology for clinical enrichment and detection analysis approved by FDA currently, which is a semi-automatic technology, integrating immunomagnetic bead sorting technology and immunocytochemical separation detection technology. Wherein, magnetic beads labeled with anti-EpCAM antibody are bound to target cells and are retained under the action of an external magnetic field, then Fluorescent labels (CK8/18/19, DAPI, CD45) are used to distinguish CTCs from blood cells, followed by analysis of cell size and morphology using a semi-automated fluorescence microscopy to ultimately identify CTCs.

However, this method is of complicated operation, high cost and low throughout, which limits its application in clinical large-scale screening and detection. In addition, due to the presence of a certain number of non-neoplastic epithelial cells (circulating epithelial cells) in the peripheral blood, and most of the vicious circulating tumor cells, due to epithelial-mesenchymal transition, lose the epithelial antigen EpCAM and thus cannot be detected. These issues are therefore particularly important when CTC counting is used to assess tumor response to treatment, risk of tumor recurrence and tumor screening. In addition, because multi-step cell labeling and processing will disperse cell clumps, immunomagnetic separation cannot be used to detect the CTMs with a higher risk of metastasis.

Another CTC enrichment method adopts the principle of density gradient separation. Depending on various densities of various cells in the blood, a solution that has a specific density and is nearly isotonic (stratified solution) is added to the same test tube with a specimen and then centrifuged so that cells with certain densities are distributed according to corresponding density gradient, making various cells in the blood separated. Further analysis such as cell staining and immune labeling can be performed after separation. The OncoQuick method (Greiner, Germany) is established on this basis, comprising using a dedicated 50 mL test tube with a built-in porous barrier, below which is a density gradient separation solution. During use, a specimen is positioned over the barrier to avoid the separation from the separation solution before centrifugation. Compared with the previous one (CellSearch system), this method is easier to isolate tumor cells from granulocytes and thereby more conducive for further analysis.

The limitation of this method lies in that some tumor cells can migrate to the plasma layer or remain in red blood cells or neutrophils, which is not only easy to cause the loss of tumor cells during separation, but also allows the tiny clots to impel tumor cells to fall to the bottom of the gradient density fluid if the blood anticoagulation is incomplete. Therefore, the method has relatively low sensitivity and depends on various factors such as the characteristics of tumor cells, centrifugation time and temperature.

In summary, the methods currently common in the related technologies for purifying circulating tumor cells have technical problems such as complicated operation, low automation degree, low throughout and low sensitivity (depending on the characteristics of tumor cells, centrifugation time, temperature, etc.).

In view of this, the present invention is hereby proposed.

### Summary of the Invention

The first object of the present invention is to provide an apparatus for high-throughput rapid trapping and purifying of circulating tumor cells, which has the advantages of high automated degree, high throughput and less possibility to cause cross contamination as used for high-throughput rapid trapping and purifying of circulating tumor cells.

The second object of the present invention is to provide a method for purifying circulating tumor cells, which is of simple operation, overcoming defects of the common circulating tumor cell purification operation in the prior art, and being capable of rapid purification of circulating tumor cells.

The third object of the present invention is to provide a method for purifying circulating tumor cells by using the apparatus for high-throughput rapid trapping and purifying of circulating tumor cells, which is of simple operation, overcoming a potential threat to the experimenters in the common circulating tumor cell purification operation in the prior art that mostly relies on manual operations.

In order to achieve the above objects of the present invention, the following technical solutions are adopted:
The present invention provides an apparatus for high-throughput rapid trapping and purifying of circulating tumor cells, comprising: a filter module; the filter module comprises a filter, a filter membrane which is arranged inside the filter and sealed with an inner wall of the filter, and a negative pressure device. The negative pressure device is connected with an end of an outlet of the filter, and used for maintaining a negative pressure within the filter; when a negative pressure is maintained within the filter by the negative pressure device, the filter membrane is configured to trap circulating tumor cells.

The apparatus for high-throughput rapid trapping of circulating tumor cells provided by the present invention comprises a filter module, the filter module comprising a filter, a filter membrane which is arranged inside the filter and sealed with an inner wall of the filter, and a negative pressure device connected with an end of an outlet of the filter. In the process of trapping circulating tumor cells, the sample liquid is input into the filter body in advance. The negative pressure device can form a negative pressure within the filter, and allows the sample liquid to pass through the filter membrane and be discharged from the outlet end of the filter. In the process of passing the sample liquid through the filter membrane, the circulating tumor cells contained in the sample liquid are trapped by the filter membrane due to their larger size than other blood cells, thereby achieving high-throughput rapid trapping of circulating tumor cells. As the apparatus may automatically control loading a sample liquid to the filter as well as the negative pressure device, the process in which the circulating tumor cells are trapped by using the filter membrane has the advantages of high automated degree and high throughput (which can be achieved by means of increasing the number of filters). Moreover, when multiple filters are operated at the same time, each filter and pipeline through which the filtrate flows is an independent pipeline, without occurring cross-contamination, and the filtrate can be collected separately for other analysis or directly discharged into the waste liquid collecting device.

Optionally, the apparatus for high-throughput rapid trapping and purifying of circulating tumor cells further comprises: a reagent loading module in connection with the filter.

Optionally, the apparatus for high-throughput rapid trapping and purifying of circulating tumor cells further comprises: a first negative pressure pump disposed on the connecting pipeline between the reagent loading module and the filter.

After the filtration of the sample liquid in the filter, the circulating tumor cells trapped on the filter membrane are generally subjected to operations, such as washing and staining, to meet the requirements of subsequent experimental analysis. Therefore, in order to further improve the automation degree of washing and staining, it is preferred to provide the reagent loading module and the first negative pressure pump. During use, under the action of the first negative pressure pump, the reagent in the reagent loading module can be smoothly introduced into the filter, thereby facilitating the washing and staining operation of the sample liquid.

Optionally, a plurality of the filters are provided and one reagent dispenser is disposed on the connecting pipeline between each of the filters and the first negative pressure pump.

In order to achieve the effect of batch processing of sample fluids, a plurality of the filters may be preferably provided and one reagent dispenser is disposed on the connecting pipeline between each of the filters and the first negative pressure pump; thereby the reagents input by the sample loading module are distributed into multiple filters by the reagent dispenser. More preferably, the plurality of filters (typically 8-10) can be formed as a set of fixed filters in an integral design through a fixing bracket. When the amount to be processed is relatively large, a plurality of sets of filters can be disposed in parallel.

For the structure of the filter, the filter preferably comprises a tube body, a sealing ring, and a bracket for placing the filter membrane, the filter membrane being disposed on the filter membrane bracket, and the sealing ring allowing the filter membrane, the filter membrane bracket and the inner wall of the tube body sealed.

Optionally, the reagent loading module comprises a washing liquid storage bottle, a staining liquid storage bottle and a preserving liquid storage bottle; the washing liquid storage bottle, the staining liquid storage bottle and the preserving liquid storage bottle are connected to the first negative pressure pump.

For example, the washing liquid storage bottle, the staining liquid storage bottle and the preserving liquid storage bottle may be connected to the first negative pressure pump through one multi-channel connector.

In general, the trapped circulating tumor cells after filtration are often subjected to operations of washing, staining and sealed preservation (if not used immediately). Therefore, the reagent loading module preferably comprises a washing liquid storage bottle, a staining liquid storage bottle and a preserving liquid storage bottle; and preferably, the washing liquid storage bottle, the staining liquid storage bottle and the preserving liquid storage bottle are connected to the first negative pressure pump through one multi-channel connector (to facilitate the connection between the plurality of storage bottles and the reagent dispenser).

Optionally, channel valves are respectively disposed on the connecting pipelines from the washing liquid storage bottle, the staining liquid storage bottle and the preserving liquid storage bottle to the multi-channel connector.

The flow direction of the reagents inside the plurality of storage bottles can be conveniently controlled through the multi-channel valves, further increasing the degree of automation.

Optionally, a waste liquid collecting device is further included, which is in connection with the outlet of the filter; and an overflow port is further disposed on the pipe wall of the filter and connects with the waste liquid collecting device through an overflow pipe.

Optionally, the apparatus further comprises a second negative pressure pump, which is disposed on the overflow pipe.

As the outlet of the filter is in connection with the waste liquid collecting device, the filtered sample liquid and the reagent flowing out of the storage bottles can be collected by the waste liquid collecting device after use. Moreover, due to the disposition of the overflow pipe and the second negative pressure pump (preferably a peristaltic pump or a vacuum pump), the second negative pressure pump is started at the beginning of the operation, thus actively preventing the sample liquid and the reagent liquid to overflow due to the clogging of the filter membrane, which maximally avoids contamination between samples during high-throughput operation, and simultaneously avoids the adverse effects of toxic and hazardous substances on instruments and operators.

Optionally, the apparatus further comprises a programmable logic controller;
the programmable logic controller is connected with all of the channel valves, the negative pressure device, the first negative pressure pump, the second negative pressure pump, the multi-channel connector and the reagent dispenser, and is used to control on/off states of the channel valves, the negative pressure device, the first negative pressure pump, the second negative pressure pump, the multi-channel connector and the reagent dispenser.

Controlling the on/off states of the channel valves, the negative pressure device, the first negative pressure pump, the second negative pressure pump, the multi-channel connector and the reagent dispenser by the programmable logic controller further increases the automation degree in the entire process for trapping circulating tumor cells, reduces the errors that can be possibly caused by manual operations as well as the potential risks that may be caused by hazards of the reagents to the human body.

Optionally, the cross-sectional area of a filter hole in the filter membrane remains unchanged along the axial direction of the filter holes, or decreases along the axial direction of the filter holes, or decreases first and then increases along the axial direction of the filter holes.

Preferably, the filter hole is a straight hole or a tapered hole or a double tapered hole; preferably, the filter hole is a double tapered hole.

Preferably, the filter hole has a hole size of 5-10 µm.

The straight hole preferably has a diameter of 8-10 µm, the tapered hole preferably has a diameter of 8-10 µm at the large opening end and a diameter of 5-7 µm at the small opening end, the double tapered holes preferably has a minimum diameter of 5-7 µm and a maximum diameter of 8-10 µm.

Optionally, the filter membrane is a polymeric microporous membrane (more specifically, for example, a polycarbonate membrane, a polyester membrane; and production mode for the hole size preferably adopts an etching method), preferably, the polymeric microporous membrane has a hole diameter of 5-10 µm.

For the outlet end of the filter connected with the negative pressure device, it is preferably provided with a tapered filter head, and the filter head of each filter is connected to the filtrate collecting tube or the waste collecting device through a separate filtrate delivery tube. If the filtrate is collected in the filtrate collection tube, since each sample and each batch of samples are operated independently, the cross-contamination between the same batch of samples and between different batches of samples is maximally avoided, thus the filtrate in the fluid collecting tube can still be used for other experimental analyses. In addition, the filter membrane is preferably a transparent or semitransparent polymeric microporous membrane, and may be a polymeric microporous membrane having a black non-fluorescent background, and the microporous membrane has an average hole diameter of 5-10 µm, while the flow of the sample liquid or reagents in the filter is realized through a negative pressure formed by the negative pressure device (preferably a peristaltic pump, a vacuum pump, a plunger pump, a syringe pump), and a 5-10 µm polymeric microporous membrane can achieve excellent trapping of circulating tumor cells.

The present invention also provides a method for purifying circulating tumor cells, comprising: passing a blood sample through a filter membrane under a negative pressure condition to trap circulating tumor cells in the blood sample on the filter membrane.

The cells with smaller size in a blood sample can pass through the filter membrane rapidly under the negative pressure condition. The method is of simple operation, overcomes the defects of the common circulating tumor cell purification operation in the prior art, and can achieve rapid purification of circulating tumor cells.

Preferably, the method comprises washing the tumor cells trapped on the filter membrane, staining, and then preserving the resulting cells in a preserving liquid.

In some embodiments of the present invention, the blood sample is subjected to cell fixation before passing through the filtration membrane. Preferably, the cell fixation is carried out by adding a paraformaldehyde solution to the blood sample. Preferably, the paraformaldehyde solution has a concentration of 4 wt%.

A method for purifying circulating tumor cells by using the apparatus for high-throughput rapid trapping of circulating tumor cells, comprising the following steps:
delivering a blood sample into the filter, starting the negative pressure device to form a negative pressure within the filter, and thus, during passing the blood sample through the filter membrane, tumor cells contained therein are trapped on the filter membrane.

Preferably, the method specifically comprises the following steps:
1) fixing the sample liquid, followed by adding into the filter, starting the negative pressure device to allow the sample liquid to pass through the filter membrane and the circulating tumor cells contained therein to be trapped; and
2) washing the circulating tumor cells trapped on the filter membrane with a phosphate buffer, staining with a staining liquid, washing with another phosphate buffer, and preserving with a preserving liquid in sequence, and obtaining the product.

Wherein, preferably, the staining liquid is any one selected from the group consisting of a DAPI, an AO, a PI, a hoechst, a syb green, a fluorescent-labeled antibody, a eosin-methylene blue and a Wright-Gemsa.

The method realizes the trapping and staining of circulating tumor cells by means of negative pressure suction filtration, and the entire operation is simple, practicable and high controllable.

As compared to the prior art, the present invention has the following beneficial effects:
(1) The overall structure of the apparatus is coherent and compact, which provides a guarantee for rapid and efficient trapping of circulating tumor cells. Based on the structural advantages of the device, high automated trapping of circulating tumor cells can be achieved.
(2) The operations such as separating, washing, staining and preserving the circulating tumor cells can be realized by automatically controlling the whole apparatus, which overcomes the defects such as potential harm to the experimenter due to the requirement of a large number of manual operations which may exist in the prior art, and low trapping efficiency.
(3) Due to the special design of the filter, pipeline and negative pressure device, each sample and each batch of sample are operated independently, which maximally avoids cross-contamination between the same batch of samples and different batches of samples, thus the re-collected filtrate can still be used for other experimental analysis, which greatly expands the application range of the apparatus, making it possible to perform multiple detection analysis on the same tube of sample.
(4) The combination of the apparatus and the method overcomes the defects of low throughput, large error, easy availability to cause cross-contamination, and especially easy availability to cause interference in subsequent molecular biological detection, existing in the common operation of manually trapping circulating tumor cells in the prior art.

### Brief Description of the Drawings

In order to illustrate the embodiments of the present invention or the technical solutions in the prior art more clearly, the drawings used in the embodiments or the description on the prior art will be briefly described below.

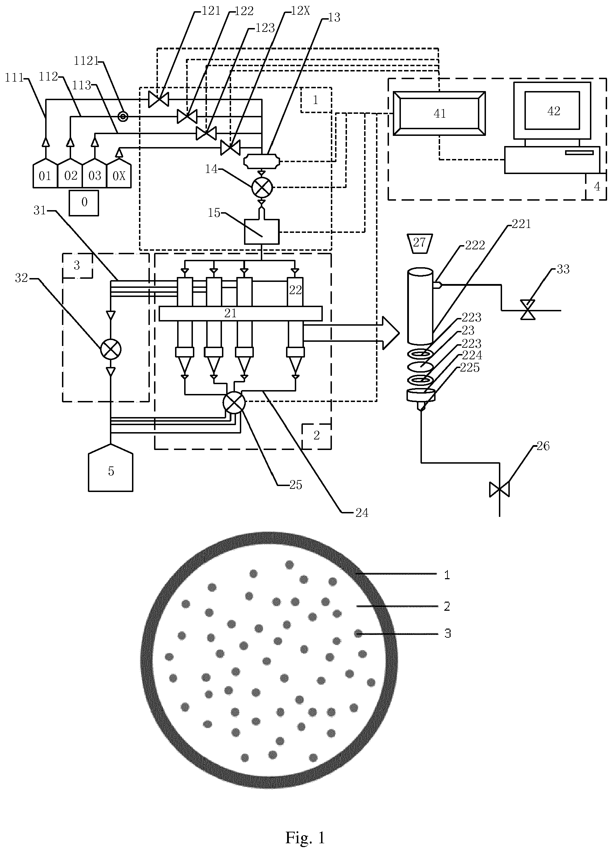

Fig. 1 is a schematic view showing the structure of an apparatus for high-throughput rapid trapping of circulating tumor cells provided by the present invention;
   Reference numerals:
   0-reagent storage bottle, 1-reagent loading module, 2-filter module, 3-active anti-overflow module, 4-automatic control module, 5-waste liquid collecting device; 01 is a washing liquid storage bottle, 02 is a staining liquid storage bottle, 03 is a preserving liquid storage bottle, 0X is a expandable reagent storage bottle, 111-1IX are reagent delivery pipes, 121-12X are channel valves, 13 is a multi-channel connector, 14 is a first negative pressure pump, 15 is a reagent dispenser; 21 is a filter fixing bracket, 22 is a filter, 23 is a filter membrane, 24 is a filtrate delivery pipe, 25 is a negative pressure device, 26 is a filtrate delivery pipe sealing clip, 27 is a sealing plug, 221 is a pipe body, 223 is a sealing ring, 224 is a bracket for placing the filter membrane, 225 is a filter head, 222 is an overflow port, 31 is an overflow pipe, 32 is a second negative pressure pump, 41 is a programmable logic controller, 42 is a control panel;
Fig. 2 is a graph showing the results of trapping and purifying of circulating tumor cells by using the apparatus of the present invention;
Fig. 3 is a structural view of a filter membrane provided in an embodiment of the present invention.

### Detailed Description

The embodiments of the present invention will be described in detail below with reference to the examples, however, those skilled in the art will understand that the following examples are merely illustrative of the invention and are not intended to limit the scope of the invention. Conditions in Examples which are not specified are carried out according to conventional conditions or conditions recommended by the manufacturer. The reagents or instruments without the specified preparation manufacturer are all conventional products that are commercially available.

### Example 1

A method for purifying circulating tumor cells, comprising:
a blood sample was passed through a filter membrane under a negative pressure condition, allowing circulating tumor cells in the blood sample to be trapped on the filter membrane. The negative pressure condition can be provided by equipments such as a vacuum pump, and the cells with smaller size in a blood sample can pass through the filter membrane rapidly under the negative pressure condition. The cells with larger size cannot pass through the filter membrane and are thus trapped on the filter membrane.

Wherein, the filter membrane is preferably a polymeric microporous membrane (more specifically, for example, a polycarbonate membrane, a polyester membrane; the hole production mode thereof is preferably an etching method), preferably, the polymeric microporous membrane has a hole diameter of 5-10 µm.

As a preferred embodiment, the blood sample is subjected to cell fixation before filtration. For example, the cell fixation was carried out by adding a paraformaldehyde solution to the blood sample. As a preferred embodiment of the example:
3 ml of paraformaldehyde solution (preferably a 4-8 wt% paraformaldehyde solution, more preferably a 4 wt% paraformaldehyde solution) was added to each 2 ml of a peripheral blood sample, and treated for 10 minutes to fix the cells in the peripheral blood and inactivate the possible pathogenic microorganisms at the same time.

After the cells were fixed, the blood sample was passed through the filter membrane under a negative pressure condition, and after the filtration was completed, the tumor cells trapped on the filter membrane are washed, and the washing liquid may be a phosphate buffer (with a pH of 7.4), a physiological saline, a glucose solution (5 wt%) and the like. When washing, the washing liquid also passed through the filter under the negative pressure condition, so that the washing liquid can act to wash the filter membrane and the cells trapped thereon.

Staining was conducted after the washing was completed, and in this example, the staining liquid was a Wright-Gemsa staining liquid (the type of the staining liquid was selected according to the actual situation), and the staining was also carried out under a negative pressure condition, so that the staining liquid can pass through the filter membrane and act to stain the cells on the filter.

The staining liquid may also be a nucleus or/and endochylema fluorescent staining liquid, preferably a staining liquid such as DAPI, AO, PI, hoechst, syb green, or various eukaryotic cell staining liquids, such as eosin-methylene blues staining liquid.

Alternatively, in order to further distinguish between leukocytes and circulating tumor cells on the filter membrane, the circulating tumor cells on the filter may be negatively stained with a fluorescently labeled CD45 antibody. The staining method is the same as the above steps.

After the staining was completed, the washing may be performed again with a phosphate buffer (with a pH of 7.4), a physiological saline, a glucose solution (5 wt%) and the like, and the washing times can be freely selected, preferably 2 times.

The tumor cells were then preserved in a preserving liquid, and the preserving liquid can be any mounting media that is soluble in water or ethanol, preferably anti-fluorescence quenching mounting liquid, glycerin, a solution in which glycerin is mixed with water or other buffer in various ratios, polyvinyl alcohol, a solution in which polyvinyl alcohol is mixed with water or other buffer mixed in various ratios, and the like.

### Example 2

Referring to Fig. 1, the apparatus for high-throughput rapid trapping of circulating tumor cells provided by the present invention comprises a filter module 2, the filter module 2 comprises a filter 22, a filter membrane 23 and a negative pressure device 25 which are arranged inside the filter 22 and which are sealed with an inner wall of the filter 22; the negative pressure device 25 is connected with an end of an outlet of the filter 22, and used for maintaining a negative pressure within the filter 22; when a negative pressure is maintained within the filter 22 by the negative pressure device 25, the filter membrane 23 is configured to trap circulating tumor cells.

In a preferred embodiment, the apparatus for high-throughput rapid trapping of circulating tumor cells further comprises: a reagent loading module 1 in connection with the filter 22, and a first negative pressure pump 14 disposed on the connecting pipeline between the reagent loading module 1 and the filter 22. A plurality of the filters 22 are preferably provided and one reagent dispenser 15 is disposed on the connecting pipeline between each of the filters 22 and the first negative pressure pump 14.

In order to further improve the automation degree of washing and staining the trapped circulating cells after filtration, it is preferred to provide the reagent loading module 1 and the first negative pressure pump 14. During use, under the action of the first negative pressure pump 14, the reagent in the reagent loading module 1 can be smoothly introduced into the filter 22, thereby facilitating the washing and staining operation of the sample liquid. In order to achieve the effect of batch processing of a sample fluid, a plurality of the filters 22 may be preferably provided and one reagent dispenser 15 is disposed on the connecting pipeline between each of filters 22 and the first negative pressure pump 14; thereby the reagents input by the sample loading module 1 are distributed into the plurality of filters 22 by the reagent dispenser 15.

Based on the above preferred embodiment, preferably, the reagent injection module 1 comprises a washing liquid storage bottle 01, a staining liquid storage bottle 02 and a preserving liquid storage bottle 03; and the washing liquid storage bottle 01, the staining liquid storage bottle 02 and the preserving liquid storage bottle 03 are connected to the first negative pressure pump so that the first negative pressure pump 14 can provide transportation power for the washing liquid in the washing liquid storage bottle 01, the staining liquid storage bottle 02 and the preserving liquid storage bottle 03.

In this example, the washing liquid storage bottle 01, the staining liquid storage bottle 02 and the preserving liquid storage bottle 03 are connected to the first negative pressure pump 14 through one multi-channel connector 13. Channel valves are respectively disposed on the connecting pipelines between the washing liquid storage bottle 01, the staining liquid storage bottle 02 and the preserving liquid storage bottle 03, and the reagent dispenser 15. The flow direction of reagents inside the plurality of storage bottles can be conveniently controlled through multi-channel valves, further increasing the automation degree of the apparatus.

Based on the above method, more preferably, the apparatus for high-throughput rapid trapping of circulating tumor cells further comprises a waste liquid collecting device 5 and a second negative pressure pump 32; the outlet of the filter 22 is in connection with the waste liquid collecting device 5; and an overflow port 222 is further disposed on the pipe wall of the filter 22 and connects with the waste liquid collecting device 5 through the overflow pipe 31; preferably, the second negative pressure pump 32 is disposed on the overflow pipe 31.

Thus, the filtered sample liquid and the reagent flowing out of the storage bottles can be collected by waste liquid collecting device 5 after use or be re-collected into the filter collecting tube. Moreover, due to the disposition of the overflow pipe 31 and the second negative pressure pump 32 (preferably a peristaltic pump, a vacuum pump), the second negative pressure pump 32 was started at the beginning of the operation, thus actively preventing the sample liquid and the reagent liquid to overflow due to the clogging of filter membrane 23, which maximally avoids the contamination between samples during high-throughput operation, while avoiding the adverse effects of toxic and hazardous substances on instruments and operators.

In addition, in order to achieve further automated control, based on the above-mentioned most preferred solution, preferably, a programmable logic controller 41 is further provided to be connected with all of the channel valves, the negative pressure device 25, the first negative pressure pump 14, the second negative pressure pump 32, the multi-channel connector 13 and the reagent dispenser 15, and is configured to control on/off states of the channel valves, the negative pressure device 25, the first negative pressure pump 14, the second negative pressure pump 32, the multi-channel connector 13 and the reagent dispenser 15. Based on the above preferred configurations, the automation degree in the entire process for strapping circulating tumor cells is further increased, and the errors that can be possibly caused by manual operations and the potential risks that can be aused by hazards of the reagents to the experimenters are reduced.

Further, in all of the above embodiments, in order to achieve trapping of circulating tumor cells in a high yield, the filter membrane is preferably a polymeric microporous membrance (a polycarbonate membrane or a polyester membrane), preferably, a polymeric microporous membrance having a hole size of 5-10 µm was selected.

Referring to Fig. 1, in another technical solution of the present invention, the apparatus for high-throughput rapid trapping of circulating tumor cells comprises: a reagent storage bottle 0, a reagent loading module 1, a filter module 2, and an active anti-overflow module 3, an automatic control module 4, a waste liquid collecting device 5;
the reagent storage bottle 0 is composed of a plurality of reagent storage bottles separately for reserving reagents required for sample processing, staining, washing, fixing and preserving; as shown in Fig. 1 (but not limited thereto), wherein 01 is a washing liquid storage bottle, 02 is a staining liquid storage bottle, 03 is a preserving liquid storage bottle, 0X is an expandable reagent storage bottle, (for example, a fixing liquid storage bottle, the preserving liquid storage bottle, and the like), and several related reagent storage bottles can be added according to needs of extraction and staining (which can be expanded to up to 10 or more storage bottles).

The reagent loading module 1 comprises: reagent delivery pipes 111-1IX, channel valves 121-12X, a multi-channel connector 13, the first negative pressure pump 14, a reagent dispenser 15. There are a plurality of reagent delivery pipes 111-1IX for connecting the reagent storage bottle 0 and the filter 22, and each of the reagent delivery pipes is provided with channel valves 121-12X respectively, and through the plurality of channel valves 121-12X, the flow of reagents in the plurality of reagent storage bottles (01-0X) can be conveniently controlled, and the automation degree is further increased.

During use, under the action of the first negative pressure pump 14, the reagents in the reagent storage bottles (01-0X) can be smoothly introduced in sequence into single filter 22 or the plurality of filters 22, thereby automatically achieving the washing and staining operation of the sample liquid. As all the steps, from trapping circulating tumor cells by the filtration of the sample fluid, to inputting various reagents to the plurality of filters 22 for washing, fixing and staining the trapped circulating tumor cells, may be automatically controlled, the whole process in which the circulating tumor cells are trapped by using the filter membrane and then stained has the advantages of being highly automated and having a high throughput (which can be achieved by means of increasing the number of filters).

The reagent loading module 1 is connected to the reagent storage bottles (01-0X), wherein each reagent enters the reagent dispenser 15 from the independent reagent delivery pipes (111-11X) via the multi-channel connector 13; the first negative pressure pump 14 (preferably a peristaltic pump, a plunger pump, a vacuum pump, a syringe pump) forms a negative pressure to push the reagent liquid flow; the channel valves (121-12X, preferably solenoid valves) are arranged on the independent reagent delivery pipes for each reagent for controlling the opening and closing times of the reagents passing through the channels; the reagent dispenser 15 has a positioning and dispensing function that evenly distributes the reagents entering the dispenser into the plurality of filters 22 of the filter module 2.

A particle filter 1121 is arranged on the staining liquid channel 112 for filtering the particles formed by crystallization in the staining liquid to maximally reduce minimize interference with the stained cells; the channel valves, the negative pressure pumps, the multi-channel connector 13 and the reagent dispenser 15 are each connected to the programmable logic controller 41 and are controlled by a programming program.

The filter module 2 comprises: a filter fixing bracket 21, a filter 22, a filter membrane 23, a filtrate delivery pipe 24, a negative pressure device 25; a set of filter fixing brackets 21 can simultaneously fix 8-10 filters 22, while a plurality of sets of filter fixing brackets 21 are conveniently added by a combination; the filter 22 is composed of a tube body 221, a sealing ring 223, a filter-attached bracket 224 and a filter head 225; the filter 22 is provided with an overflow port 222 on the tube body, and the overflow port 222 is connected to the active anti-overflow module 3 to prevent overflow of the sample and the reagent liquid caused by the clogging of the filter membrane 23.

The tube body 221 is assembled with the filter membrane 23, a bracket for placing the filter membrane 224, and a filter head 225 to form filter 22; a sealing ring 223 is used to seal the gap between the tube body 221 and the filter membrane 23, and the bracket for placing the filter membrane 224, which facilitates the subsequent negative pressure operation; the filter heads 225 of the filters 22 are each connected to the independent filtrate delivery pipe 24 to maximally avoid cross-contamination between the same batch of samples and different batches of samples; the delivery pipe 24 is finally connected to the waste liquid collecting device 5 or to other filtrate collecting tubes; the filter membrane 23 is a transparent or semitransparent polymeric microporous membrane, and can also be a polymeric microporous membrane having a black non-fluorescent background; the microporous membrane have an average hole diameter of 5-10 µm; the sample reagent was filtered and the filtrate flowed, and the negative pressure device 25 (preferably a peristaltic pump, a vacuum pump) formed a negative pressure to push the reagent liquid to flow; the negative pressure device 25 is connected to programmable logic controller 41 and is controlled by the programming program.

The active anti-overflow module 3 comprises an overflow pipe 31 and the second negative pressure pump 32. The overflow pipe 31 is connected to an overflow port 222 on the pipe body 221 of the filter 22, and finally connected to the waste liquid collecting device 5; the overflow pipe 31 is connected with the second negative pressure pump 32 (preferably a peristaltic pump, a vacuum pump), the negative pressure operation was started at the beginning of the sample operation, thus actively preventing the sample and the reagent liquid to overflow due to the clogging of the filter membrane 23, which maximally avoids contamination between samples during high-throughput operation, while avoiding the adverse effects of toxic and hazardous substances on instruments and operators; the second negative pressure pump 32 is connected to the programmable logic controller 41 and is controlled by the programming program.

The waste liquid collecting device 5 is connected with the filtrate delivery pipe 24 and the overflow pipe 31, and contains a pathogenic microorganism inactivating reagent (preferably sodium hypochlorite, an alcohol solution, and the like.) for inactivating the filtrate or overflow entering into the waste liquid collecting device, and thereby for effectively protecting the operating environment and personnel.

An automatic control module 4 is used to control the programmable logic controllers 41 of reagent loading module 1, the filter module 2 and the active anti-overflow module 3, and the programmable logic controller 4.1 can be connected to a computer or a control panel 42 for regulating each controller in sequence and time according to the programming program, or changing the operating procedure and time according to the specific requirements.

Next, in combination with the above, the following specific embodiments are exemplified for the method for purifying circulating tumor cells by using the apparatus for high-throughput rapid trapping of circulating tumor cells of the present invention:
The method for purifying circulating tumor cells provided by the present invention specifically comprises the following steps:

### 1. Filtration of blood sample (sample liquid):

a) 3 ml of physiological saline (or a phosphate buffer with a pH of 0.7 as a substitute) and 400 ul of 4% paraformaldehyde solution were added to 2-5ml of peripheral blood sample, thereafter, the resulting solution was made up with physiological saline or phosphate buffer (pH 7.4) to a final volume of 8 ml, and treated for 10 minutes to fix the cells in the peripheral blood and inactivate the possible pathogenic microorganisms at the same time.
b) the resulting solution assembled was added to the filter 22 in one portion or in portions, and the liquid level in the filter 2.2 after each addition should be lower than the lowermost end of the overflow port 222 of the filter 22;
c) the negative pressure device 25 was started and continuously operated for 10-15 minutes to allow the blood sample to pass through the filter membrane 23 under the action of negative pressure, so that the circulating tumor cells can be trapped on the membrane surface by the microporous membrane.

In this step, since the diameter of the circulating tumor cells is larger than that of the red blood cells and most blood nucleated cells, the circulating tumor cells are trapped on the surface of the membrane 23 by the microporous membrane, while the red blood cells and the blood nucleated cells passed through the microporous membrane and finally entered the waste liquid collecting device 5 via the independent filtrate delivery tube 24, and the possible pathogenic microorganisms in the filtrate were inactivated by the pathogenic microorganism inactivating reagent, or the filtrate was collected into other filtrate collecting tubes for other determination and analysis. The fixing solution can be any reagent having the function of fixing the cell sample, and is preferably an aldehyde fixing liquid such as paraformaldehyde and formaldehyde; or, preferably methanol, ethanol and an aldehyde-free fixing liquid.

### 2. Washing of the circulating tumor cells on the microporous membrane:

a) after the blood sample filtration step was completed, the second negative pressure pump 32 was immediately started (which was always turned on thereafter) and the first negative pressure pump 14 was started at the same time;
b) the washing liquid channel valve 121 was turned on, and a phosphate buffer (pH 7.4) as the washing liquid entered the washing liquid delivery pipe 111 from the washing liquid storage bottle 01 under the action of the first negative pressure pump 14, and the washing liquid passed through the multi-channel connector 13 and the reagent dispenser 15 so as to be evenly distributed into the plurality of filters 22;
c) after the first negative pressure pump 14 was turned on for 1-2 minutes, the negative pressure device 25 was turned on again to allow the cleaning liquid in the filter 22 to pass through the filter membrane for washing the filter membrane 23 and the cells trapped thereon.

Wherein, in the above steps, the washing liquid channel valve 121 and the first negative pressure pump 14 were turned on for 5 minutes, then the washing liquid channel valve 121 was closed, the first negative pressure pump 14 was stopped, while the negative pressure device 25 was turned on for a duration of 10-15 minutes until all of the washing fluid in the filter 22 was emptied.

Further, the washing fluid may also be a physiological saline, a 5% glucose solution or the like in addition to the phosphate buffer (pH 7.4).

### 3. Staining of the circulating tumor cells on the filter membrane:

a) after the washing step was completed, the negative pressure device 25 was suspended, the first negative pressure pump 14 was turned on again, meanwhile the staining liquid channel valve 122 was opened;
b) a Rehlet-Giemsa staining liquid as the staining liquid (the type of the staining liquid was selected according to the actual situation) entered the staining liquid delivery pipe 112 from the staining liquid storage bottle 02 under the action of the first negative pressure pump 14, and then passed through the particle filter 1121 and was evenly distributed into the plurality of filters 22 via the multi-channel connector 13 and the reagent dispenser 15;
c) after the negative pressure device 25 was suspended for 15-20 minutes, the negative pressure device 25 was turned on again, so that the staining liquid in the filter 22 passed through the filter membrane 23, and acted to stain the cells on the filter membrane 23 until all of the staining liquid in the filter 22 was emptied.

In the above steps, the staining liquid channel valve 122 and the first negative pressure pump 14 were turned on for 5 minutes, then the staining liquid channel valve 122 was closed, the first negative pressure pump 14 was stopped, while the negative pressure device 25 was continued to stop for 15-20 minutes after the first negative pressure pump 14 was stopped, thereafter, the negative pressure device 25 was turned on for a duration of 1-2 minutes until all of the staining liquid in the filter 22 was emptied.

It should be noted that, in addition to the Richter-Giemsa staining liquid, the staining liquid may also be a cell nucleus or/and endochylema fluorescent staining liquid, preferably a staining liquid such as DAPI, AO, PI, hoechst, syb green, or various eukaryotic cell staining liquids, such as eosin-methylene blues staining liquid.

### 4. Fluorescently labeled antibody staining of the circulating tumor cells on the filter membrane:

In order to further distinguish between leukocytes and circulating tumor cells on the filter membrane, the circulating tumor cells on the filter may be positively stained with a fluorescently labeled circulating tumor cell specific antibody, and the circulating tumor cells on the filter may be negatively stained with a fluorescently labeled CD45 antibody.
a) before or after step 3, the negative pressure device 25 was suspended, the first negative pressure pump 14 was turned on again, meanwhile the fluorescently labeled CD45 antibody staining liquid channel valve 12X was opened;
b) the fluorescently labeled labeled CD45 antibody staining liquid entered the staining liquid delivery pipe 11X from the fluorescently labeled CD45 antibody staining liquid storage bottle 0X under the action of the first negative pressure pump 14, and was then evenly distributed into the plurality of filters 22 via the multi-channel connector 13 and the reagent dispenser 15;
c) after the negative pressure device 25 was suspended for 60-90 minutes, the negative pressure device 25 was turned on again, so that the fluorescently labeled CD45 antibody staining liquid in the filter 22 passed through the filter membrane 23, and acted to antibody-labeled stain the cells on the filter membrane 23 until all of the staining liquid in the filter 22 was emptied.

In the above steps, the staining liquid channel valve 122 and the first negative pressure pump 14 were turned on for 1 minutes, then the staining liquid channel valve 12X was closed, the first negative pressure pump 14 was stopped, while the negative pressure device 25 was continued to stop for 60-90 minutes after the first negative pressure pump 14 was stopped, thereafter, the negative pressure device 25 was turned on for a duration of 1-2 minutes until all of the staining liquid in the filter 22 was emptied.

It should be noted that, in addition to the fluorescently labeled CD45 antibody staining liquid, the fluorescently labeled antibody staining liquid of this step can also be selected according to characteristics of the tissue to be analyzed or the type of cancer cells.

### 5. Step of washing after staining:

the specific operation of this step is the same as that of step 2, and washing times are two, which will not be described herein.

### 6. Step of preserving the cells on the membrane (optionally):

a) if the stained cells were not immediately used for subsequent analysis, this step can be used in order to ensure cell structure, staining results and stability of intracellular nucleic acids;
b) after the step of washing after staining was completed, the negative pressure device 25 was suspended, the first negative pressure pump 14 was turned on again, meanwhile the preserving liquid channel valve (shown by numeral 123 in the figures) was opened;
c) the preserving liquid entered the preserving liquid delivery pipe 113 from the preserving liquid storage bottle 03 under the action of the first negative pressure pump 14, and then the preserving liquid was evenly distributed into the plurality of filters 22 via the multi-channel connector 13 and the reagent dispenser 15;
d) after the preserving liquid entered the filter 22 and was capable to cover the filter membrane 23, all the negative pressure pumps and the negative pressure devices are closed, and closure was conducted with the filtrate delivery pipe sealing clip 26 and the overflow tube sealing clip 33, after the closure, the entire filter 22 was removed, and after the sealing plug 27 was inserted into the top of the filter, the filter 22 was preserved at 4-25 ° C;
e) in the above steps, the preserving liquid can be any mounting media that is soluble in water or ethanol, preferably anti-fluorescence quenching mounting solution, glycerin, a solution in which glycerin is mixed with water or other buffer in various ratios, polyvinyl alcohol, a solution in which polyvinyl alcohol is mixed with water or other buffer mixed in various ratios, and the like;
f) the cells preserved by using this step was preferred to undergo the washing step once again before the subsequent counting and purification analysis, wherein the washing liquid used may be a neutral isotonic buffer or ethanol given in the washing step so as to reduce the possibility of interference caused by the preserving liquid to the subsequent analysis.

### 7. Step of counting and purification of circulating tumor cells on the membrane:

a) in order to perform counting analysis on the circulating tumor cells, the stained filter membrane 23 was taken out from the filter 22, tiled on a clean glass slide or lining film, and then placed in a digital pathological scanning device to scan the filter membrane at an appropriate wavelength, because the stained circulated tumor cells and the nucleated blood cells in the blood have large differences in nuclear morphology and surface fluorescent labeling, which can be convenient to distinguish, thereby the results after scanning can be counting analyzed;
b) after the scanning ended, the filter membrane was placed on the lining film, and then the circulating tumor cells were laser-cut under a laser micro-cutter and collected in different centrifuge tubes separately for subsequent molecular biological analysis;
c) for the suspected nucleated blood cells around the circulating tumor cells, in order to prevent them from interfering with the molecular biological analysis of the cut circulated tumor cells (the nucleated blood cells are very likely to cause significant interference with the molecular biological analysis of the circulating tumor cells), the technology of single-beam high-power laser breakdown before cutting was innovatively used to destroy the suspected cells and nucleic acid molecules, maximally preventing non-specific amplification and interference.

### Experiment Example 1

A plurality of sample liquids were simultaneously processed by the above method for high-throughput rapid trapping of circulating tumor cells, to determine the trapping rate and parallelism of circulating tumor cells, and the specific operation methods are as follows:
S1: a predetermined amount of cervical cancer HeLa cells from 1000 human subjects were added to 2 ml of phosphate buffer, and then filtered by the method of the present invention, and 10 identical samples were simultaneously determined;
S2: after filtration, the cells on the membrane were photographed and counted by a digital pathological system and a counting analysis software, and the cell trapping rate = (counting result on the membrane / 1000) × 100%.

The results show (see Table 1) that a plurality of samples can be processed by using the apparatus and method of the present invention, with cell trapping rates of above 75% and good parallelism between samples.

**Table 1: Trapping rate and parallelism of tumor cells for a plurality of samples processed at the same time**

| **Filter No.** | **1#** | **2#** | **3#** | **4#** | **5#** | **6#** | **7#** | **8#** | **9#** | **10#** |
|---|---|---|---|---|---|---|---|---|---|---|
| **cell trapping rate** | 78.2% | 75.9% | 79.3% | 80.6% | 76.7% | 76.1% | 78.9% | 79.1% | 81.3% | 75.1% |

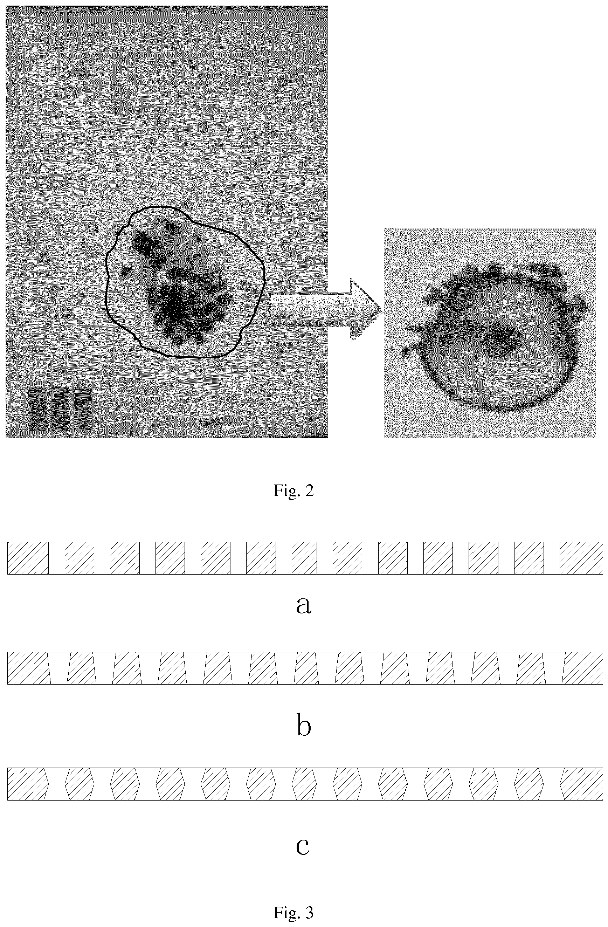

Furthermore, in Fig. 2, the results of trapping and purifying circulating tumor cells by using the apparatus of the present invention are shown: the portion circled by the black curve is the circulating tumor microembolus which was trapped on the filter membrane; the right panel is the circulating tumor microembolus which was purified by micro-cutting technology after surrounding suspected nucleated blood cells were destroyed by the technology of single-beam high-power laser breakdown before cutting; as can be seen from Fig. 2, the apparatus for purifying circulating tumor cells and the method thereof provided by the present invention achieves better separation and purification effect on circulating tumor cells.

Overall, through the apparatus and method provided by the present invention, in the process of passing the sample liquid through the filter membrane, the circulating tumor cells contained in the sample liquid are trapped by the filter membrane due to their larger size than other blood cells, thereby achieving high-throughput rapid trapping of circulating tumor cells; as the apparatus may automatically control loading a sample liquid to the filter as well as the negative pressure device (which can be preferably a negative pressure pump), the process in which the circulating tumor cells are trapped by using the filter membrane has the advantages of being highly automated and having a high throughput (which can be achieved by means of increasing the number of filters).

As a preferred embodiment of the present invention, the filter membrane in this example optionally have the filter holes with a cross-sectional area remaining unchanged along the axial direction of the filter holes, or decreasing along the axial direction of the filter holes, or decreasing first and then increasing along the axial direction of the filter holes, and referring to Fig. 3, the filter hole can be selected from, for example, a straight hole as shown in Fig. 3a, a tapered hole of as shown in Fig. 3b, or a double tapered hole of as shown in Fig. 3c, most preferably a double tapered hole. The double tapered hole described herein means that the cross-sectional areas of the two ends of the filter hole are larger than the cross-sectional area of the intermediate portion, which has a hole structure wherein both ends are wide and the middle is narrow. This double tapered hole has wonderful cell trapping and excellent filtration rate.

### Experiment Example 2

A comparison experiment of the filtration rate and the cell trapping rate of the above-mentioned membranes made up of PC material having three kinds of filtration holes was conducted, and the experimental method is as follows:
100 HepG2 hepatoma cells were added to 4 mL of whole blood from normal human, followed by filtration and staining by using the apparatus of the present invention (the difference was only in the filter membrane) according to the aforementioned test method, the filtration time was recorded, and the total number of retained cells on the membrane was counted, with 5 sheets test for each hole type of membranes; and the results show that the whole blood filtration time: straight-hole type <double tapered hole<tapered hole; for hepatocarcinoma cell trapping rate: double tapered hole> tapered hole> straight-hole type (see Table 2 for details); the results show that the membrane in double tapered hole type has a faster filtration speed and the cell trapping rate is significantly improved.

**Table 2: comparison result of filtration rates and cell trapping rates of membranes in different hole types**

| | Filter time (seconds) | HepG2 cell trapping rate (%) |
|---|---|---|
| Straight-hole membrane | 207.8±28.6 | 76.6±4.9 |
| Single tapered holes | 286.6±22.1 | 79.0±3.4 |
| Double tapered holes | 225.4±17.3 | 87.0±4.3 |

As a preferred embodiment, the filter holes can be produced by a method such as an etching method and a laser perforation method which can achieve the same hole type conditions.

More preferably, when the filter membrane in the above three types of filter holes is selected, for the inner diameter (diameter) of the filter hole,
the straight holes have a diameter of preferably 8-10 µm, the tapered holes have a diameter of preferably 8-10 µm at the large opening end and a diameter of preferably 5-7 µm at the small opening end. The double tapered holes has a wide hole diameter range of preferably 8-10 µm and a middle narrow hole diameter range of preferably 5-7 µm; and the wide hole here refers to the portion of the double tapered hole having the largest hole diameter, that is, the maximum hole diameter of the double tapered holes is preferably 8-10 µm, while the narrow hole refers to the portion of the double tapered hole having the smallest hole diameter, that is, the minimum hole diameter of the double tapered hole is preferably 5-7 µm.

It should be noted that the above embodiments are merely illustrative of the technical solutions of the present invention, and are not intended to be limiting; although the present invention has been described in detail with reference to the foregoing embodiments, it should be understood by those skilled in the art that the technical solutions described in the foregoing embodiments may be modified, or some or all of the technical features may be equivalently replaced; and these modifications and substitutions do not depart from the scope of the technical solutions of the embodiments of the present invention.

## Claims

1. An apparatus for high-throughput rapid trapping and purifying of circulating tumor cells, **characterized in** comprising: a filter module;
the filter module comprises a filter, a filter membrane which is arranged inside the filter and sealed with an inner wall of the filter, and a negative pressure device; the negative pressure device is connected with an end of an outlet of the filter, and used for maintaining a negative pressure within the filter; when a negative pressure is maintained within the filter by the negative pressure device, the filter membrane is configured to trap circulating tumor cells.

2. The apparatus for high-throughput rapid trapping of circulating tumor cells according to claim 1, **characterized in** further comprising: a reagent loading module in connection with the filter.

3. The apparatus for high-throughput rapid trapping of circulating tumor cells according to claim 2, **characterized in** further comprising: a first negative pressure pump disposed on the connecting pipeline between the reagent loading module and the filter.

4. The apparatus for high-throughput rapid trapping of circulating tumor cells according to claim 2 or 3, **characterized in that** a plurality of the filters are provided and one reagent dispenser is disposed on the connecting pipeline between each of the filters and the first negative pressure pump.

5. The apparatus for high-throughput rapid trapping of circulating tumor cells according to any of claims 2-4, **characterized in that** the reagent loading module comprises a washing liquid storage bottle, a staining liquid storage bottle and a preserving liquid storage bottle;
the washing liquid storage bottle, the staining liquid storage bottle and the preserving liquid storage bottle are connected to the first negative pressure pump.

6. The apparatus for high-throughput rapid trapping of circulating tumor cells according to claim 5, **characterized in that** the washing liquid storage bottle, the staining liquid storage bottle and the preserving liquid storage bottle are connected to the first negative pressure pump through one multi-channel connector.

7. The apparatus for high-throughput rapid trapping of circulating tumor cells according to claim 5 or 6, **characterized in that** channel valves are respectively disposed on the connecting pipelines between the washing liquid storage bottle, the staining liquid storage bottle and the preserving liquid storage bottle, and the multi-channel connector.

8. The apparatus for high-throughput rapid trapping of circulating tumor cells according to any of claims 1-7, **characterized in** further comprising a waste liquid collecting device;
an outlet of the filter is in connection with the waste liquid collecting device; and an overflow port is further disposed on the pipe wall of the filter and connects with the waste liquid collecting device through an overflow pipe.

9. The apparatus for high-throughput rapid trapping of circulating tumor cells according to claim 8, **characterized in** further comprising a second negative pressure pump disposed on the overflow pipe.

10. The apparatus for high-throughput rapid trapping of circulating tumor cells according to claim 9, **characterized in** further comprising a programmable logic controller;
the programmable logic controller is electrically connected with all of the channel valves, the negative pressure device, the first negative pressure pump, the second negative pressure pump, the multi-channel connector and the reagent dispenser, and is used to control on/off states of the channel valves, the negative pressure device, the first negative pressure pump, the second negative pressure pump, the multi-channel connector and the reagent dispenser.

11. The apparatus for high-throughput rapid trapping of circulating tumor cells according to any of claims 1-10, **characterized in that** the cross-sectional area of a filter hole of the filter membrane remains unchanged along the axial direction of the filter hole, or decreases along the axial direction of the filter hole, or decreases first and then increases along the axial direction of the filter hole;
preferably, the filter hole is straight hole or tapered hole or double tapered hole; preferably, the filter hole is double tapered hole;
preferably, the filter hole has a hole size of 5-10 µm;
the straight hole has a diameter of preferably 8-10 µm, the tapered hole has a diameter of preferably 8-10 µm at the large opening end and a diameter of preferably 5-7 µm at the small opening end, the double tapered hole has a minimum diameter of preferably 5-7 µm and a maximum diameter of preferably 8-10 µm.

12. The apparatus for high-throughput rapid trapping of circulating tumor cells according to any of claims 1-11, **characterized in that** the filter membrane is preferably a polymeric microporous membrane, preferably, the filter membrane is a polycarbonate membrane or a polyester membrane or a polyethylene membrane.

13. A method for purifying circulating tumor cells, **characterized in** comprising the following step: passing a blood sample through a filter membrane under a negative pressure condition to trap circulating tumor cells in the blood sample on the filter membrane.

14. The method for purifying circulating tumor cells according to claim 13, **characterized in** further comprising: washing the tumor cells trapped on the filter membrane, staining, and then preserving the resulting cells in a preserving liquid.

15. The method for purifying circulating tumor cells according to claim 13 or 14, **characterized in that** the blood sample is subjected to cell fixation before passing through the filter membrane.

16. The method for purifying circulating tumor cells according to claim 15, **characterized in that** the cell fixation is carried out by adding a paraformaldehyde solution to the blood sample.

17. The method for purifying circulating tumor cells according to claim 16, **characterized in that** the paraformaldehyde solution has a concentration of 4-8 wt%.

18. A method for purifying circulating tumor cells by using the apparatus for high-throughput rapid trapping of circulating tumor cells according to any of claims 1-12, **characterized in** comprising the following steps:
delivering a blood sample into the filter, starting the negative pressure device to form a negative pressure within the filter, and thus, during passing the blood sample through the filter membrane, tumor cells contained therein are trapped on the filter membrane.

19. The method for purifying circulating tumor cells by using the apparatus for high-throughput rapid trapping of circulating tumor cells according to claim 18, **characterized in** specifically comprising the following steps:
1) fixing the sample liquid, followed by adding into the filter, starting the negative pressure device to allow the sample liquid to pass through the filter membrane and the circulating tumor cells contained therein to be trapped;
2) washing the circulating tumor cells trapped on the filter membrane with a phosphate buffer, staining with a staining liquid, washing with another phosphate buffer, and preserving with a preserving liquid in sequence, and obtaining the product.

20. The method for purifying circulating tumor cells by using the apparatus for high-throughput rapid trapping of circulating tumor cells according to claim 19, **characterized in that** the staining liquid is any one selected from the group consisting of a DAPI, an AO, a PI, a hoechst, a syb green, a fluorescent-labeled antibody, a eosin-methylene blue, a Wright-Gemsa, a HE staining liquid, a neutral red staining liquid.
